# EUROPEAN PATENT APPLICATION

(11) **EP 2 022 426 A2**
(43) Date of publication of application: **11.02.2009**
(21) Application number: 08013839.9
(22) Date of filing: 01.08.2008
(51) Int. Cl.: A61B 17/88

(54) **Distribution device applicable to vertebroplasty dosing**

(30) Priority: 02.08.2007 IT PD20070266
(71) Applicant: Facco, Michela, 35123 Padova (IT)
(72) Inventor: Facco, Michela, 35123 Padova (IT)
(74) Representative: Vinci, Marcello

(57) **Abstract**

This is a new device for attaching to manual dosing devices for use in vertebroplasty, comprising a body (1) fitted with a coupling or connector (2) for connecting it to a manual dosing device, and two or more delivery tubes (3), each with an applicator needle, wherein said body (1) comprises a connection chamber (1.1) placing said connector (2) and said tubes (3) in communication via said body (1), and wherein a diverter or conveyor element (4), with passages (4.1), contained inside said chamber (1.1) in said body (1), and controllable from the outside of said body (1), places said connector (2) in communication with one or more of the delivery tubes (3), depending on the position in which said element is placed. The passages (4.1) through the diverter or conveyor element (4) are all in communication with one another.

## Description

The present patent relates to medical fluid dosing devices and applicators, and particularly those for delivering cementing substances, as used in vertebroplasty.

Some diseases of the spinal column are treated with intersomatic spinal fusion, or more in general with a vertebroplasty, to restore sagittal balance and improve the condition of the spine as a whole.

These procedures often involve the injection of cementing substances through specific devices for the dose-controlled delivery of medical fluids, and particularly of setting resins of the bone cement type.

There are known manual devices for dosing medical fluids, such as setting resins of the bone cement type, that comprise a chamber containing the fluid to deliver that has a connector for the delivery tube at one end and a plunger at the other open end. The plunger is capable of translation inside the chamber, through said open end, and is fitted with operating means for the user and a screw-shaped member inserted between said operating means and said plunger so as to enable its longitudinal movement, which pushes the fluid being delivered.

A constraint/release mechanism engages or releases the threaded part of said screw member.

By taking action on said constraint/release mechanism, the user can either engage or release said threaded member.

When the threaded member is engaged, its translation can only take place as a consequence of its rotation; when the threaded member is released, the user can take action directly to translate said threaded member and the corresponding plunger.

All the manual devices for dosing medical fluids used in vertebroplasty have only one connector for the delivery tube connected to the applicator needle.

One of the necessary characteristics of the medical fluids used in vertebroplasty is a relatively rapid solidification.

The quantity of fluid to inject varies, depending on the patient's condition and on the site of application or access route.

The operator is obliged to prepare a greater quantity of fluid than is expected to be needed, however, to allow for the product needed to fill the applicator needle and delivery tube, plus some spare product in case a larger quantity of fluid than expected proves necessary during the procedure.

On completion of an application, all the fluid remaining inside the needle, the delivery tube and the dosing device cannot be reused and is discarded.

A single application at a specific site is not always enough to achieve intersomatic spinal fusion, or a vertebroplasty in general, in order to deal with the patient's condition.

It often becomes necessary to perform several applications at two or more different sites, or from two different angles at the same site or within the same vertebra. This means that the operator has to prepare and use two applicator needles and corresponding delivery tubes and two distinct manual dosing devices, each containing its own quantity of fluid. This makes it necessary to have two or more manual dosing devices already loaded with the required amount of fluid and time enough to inject the product before it loses its fluid properties and begins to become pasty and solidify.

Moreover, the fluid application involved in vertebroplasty is not a continuous, uninterrupted procedure, but may involve two or more uses of the same manual dosing device, depending on the patient's condition and the situation in hand, separated by a brief interval to make adjustments and check the penetration of the fluid.

The operator is consequently obliged to handle several manual dosing devices, one of which is being used and the others are awaiting further use, becoming a hindrance and a cause of confusion during the procedure.

A manual dosing device cannot be disconnected from a delivery tube to connect it to another delivery tube because it is essential to maintain a constant pressure on the fluid contained in the delivery tubes and also to avoid any air leaking inside said tubes.

To overcome the above-mentioned drawbacks, a new dispenser has been designed and realised for use with the manual dosing devices used in vertebroplasty.

One object of the new dispenser is to enable the connection of a single manual dosing device to at least two delivery tubes and their respective applicator needles.

Another object of the new dispenser is to enable the user to choose which of the delivery tubes connected is to receive the fluid placed under pressure in the dosing device.

Another object of the new dispenser is to maintain the pressure in each delivery tube even when the dosing device is not connected to said delivery tube.

Another object of the new dispenser is to reduce the number of manual dosing devices needed for the application of the fluid at two or more sites or via different access routes.

Another object of the new dispenser is to enable the preparation and use of a single dosing device without having to wait or prepare a second dosing device.

These and other direct and complementary objects are achieved by the new dispenser for attaching to manual dosing devices for use in vertebroplasty and comprising a body fitted with a coupling or connector for connecting to a manual dosing device and to two delivery tubes with respective applicator needles, wherein said body forms a connection chamber that places in communication said connector and said delivery tubes on said body. A diverter or conveyor element, contained inside said connection chamber in said body, and controllable from the outside of said body, alternately places said connector attached to the manual dosing device in communication with one of the delivery tubes and the corresponding applicator needles, depending on the position in which said diverter element is placed.

The operator prepares the applicator needles and the manual dosing device, then connects the new dispenser between the dosing device and the applicator needles.

The operator then uses the manual dosing device and, depending on which needle is to be used to deliver the fluid, he shifts the lever or wheel, or other means provided on the new dispenser, to position the diverter or conveyor element suitably so as to place the manual dosing device in communication with the chosen delivery tube and the applicator needle.

The new dispenser for attaching to manual dosing devices for use in vertebroplasty is designed so that the diverter or conveyor element places said connector in communication with one and only one of the delivery tubes, and that the other delivery tube(s) are simultaneously completely sealed off.

Said diverter or conveyor element can also be arranged in a neutral position, i.e. in a position where there is no communication via the connector between the dosing device and any of the delivery tubes.

The characteristics of the new dispenser for attaching to a manual dosing device for use in vertebroplasty are better clarified in the following description, which refers to the drawings that are attached hereto as a nonlimiting example.

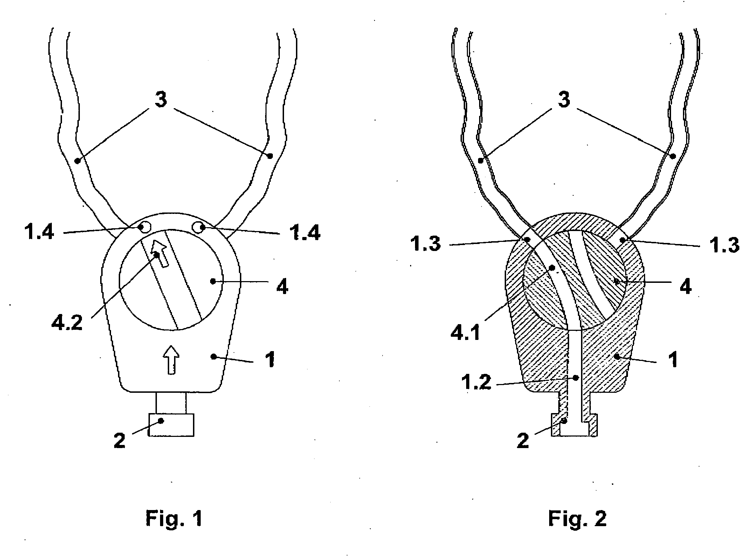

Figure 1 shows an outside view of the new dispenser, while figure 2 is schematic view of the inside of the new dispenser.

The new dispenser for attaching to a manual dosing device for use in vertebroplasty comprises a body (1) fitted with a coupling or connector (2) for its connection to a manual dosing device, and two delivery tubes (3) with corresponding applicator needles.

Said body (1) contains a connection chamber (1.1) which places in communication said connector (2) and said delivery tubes (3) on said body (1).

A diverter or conveyor element (4) contained inside said chamber (1.1) in said body (1), and controllable from the outside of the body, alternately places said connector (2) to the manual dosing device in communication with one of the delivery tubes (3) and the corresponding applicator needles, depending on the position in which said diverter element is placed.

In this example, the chamber (1.1) is circular in shape and is connected by means of passages (1.2, 1.3) with the connector (2) and the delivery tubes (3).

The diverter or conveyor element (4) is also circular and generically cylindrical in shape, and designed to fit exactly inside said chamber (1.1). In particular, said diverter or conveyor element (4) extends from the upper part of the body (1) and it has through holes or passages (4.1) in the portion contained inside the chamber (1.1), so as to place the passage (1.2) from the connector (2) in communication with one and only one passage (1.3) leading to a delivery tube (3).

The through holes or passages (4.1) in the diverter or conveyor element (4) may be in communication with one another, or they may be separate (as in the example) so that each through hole or passage (4.1) can place the passage (1.2) from the connector (2) in communication with one passage (1.3) towards a delivery tube (3).

Signalling means (1.4, 4.2) are provided to indicate which delivery tube (3) is currently in communication with the connector (2).

The operator prepares the applicator needles and the manual dosing device, then connects the new dispenser to the dosing device and to the applicator needles.

The fluid delivered by the dosing device is channelled through the new dispenser to one or other of the delivery tubes (3) and applicator needles, depending on the position of the diverter or conveyor element (4).

A simple action by the operator on said diverter or conveyor element (4) enables the fluid to be sent from the same dosing device to another delivery tube (3) and another applicator needle. The tube (3) that is disconnected from the dosing device is closed, thus becoming insensitive to the action of the dosing device and airtight. The pressure of the fluid inside said disconnected tube (3) remains unchanged.

Thus, with reference to the above description and the attached drawings, the following claims are advanced.

## Claims

1. A device for attaching to manual dosing devices for use in vertebroplasty, **characterized in that** it comprises a body (1), complete with a coupling or connector (2) designed to be connected to a manual dosing device and to two or more delivery tubes (3), each with a respective applicator needle, wherein said body (1) has a connection chamber (1.1) that places said connector (2) in communication with said tubes (3) via said body (1), and wherein a diverter or conveyor element (4), with passages (4.1), contained inside said chamber (1.1) in said body (1), and controllable from the outside of said body (1), alternately places said connector (2) to the manual dosing device in communication with one or other of the delivery tubes (3) leading to the applicator needles, depending on the position in which said diverter element (4) is placed.

2. A device for attaching to manual dosing devices for use in vertebroplasty according to claim 1, **characterized in that** the passages (4.1) through the diverter or conveyor element (4) are all in communication with one another.

3. A device for attaching to manual dosing devices for use in vertebroplasty according to claim 1, **characterized in that** it comprises separate passages (4.1) for each delivery tube (3).

4. A device for attaching to manual dosing devices for use in vertebroplasty according to the previous claims, **characterized in that** it includes signalling means (1.4, 4.2) on the outside of the diverter or conveyor element (4) to indicate which delivery tube (3) is currently in communication with the connector (2).

5. A device for attaching to manual dosing devices for use in vertebroplasty according to the previous claims, **characterized in that** said diverter or conveyor element (4) is fitted with locking or safety means for retaining it in a neutral position wherein there is no communication between the connector (2) to the dosing device and any of the delivery tubes (2).
